(19) Europäisches Patentamt

European Patent Office

Office européen des brevets

(11) EP 0 747 046 A2

(12) **EUROPEAN PATENT APPLICATION**

(43) Date of publication:
11.12.1996 Bulletin 1996/50

(51) Int Cl.⁶: **A61K 9/22**, A61F 2/02

(21) Application number: 96303400.4

(22) Date of filing: 14.05.1996

(84) Designated Contracting States:
**DE FR GB IT**

(30) Priority: **19.05.1995 US 446207**

(71) Applicant: **BAXTER INTERNATIONAL INC.**
**Deerfield, Illinois 60015 (US)**

(72) Inventors:
• **Sternberg, Shmuel**
  **Northbrook, Illinois 60062 (US)**
• **Pauley, Robin G.**
  **Ingleside, Illinois 60041 (US)**
• **Boggs, Daniel R.**
  **Libertyville, Illinois 60048 (US)**
• **McLarty, Donna L.**
  **Hoffman Estates, Illinois 60195 (US)**
• **Khare, Atul R.**
  **Crystal Lake, Illinois 60014 (US)**
• **Brauker, James H.**
  **Lake Villa, Illinois 60046 (US)**
• **Martinson, Laura A.**
  **Seattle, Washington 98107 (US)**

(74) Representative: **MacGregor, Gordon et al**
**ERIC POTTER CLARKSON**
**St. Mary's Court**
**St. Mary's Gate**
**Nottingham, NG1 1LE (GB)**

(54) **Permeable immunoisolation membrane structures for implantation of cells in host tissue**

(57)    Permeable structures suited for implanting in host tissue include a first permeable region comprising a polymer solution formed-in-place in contact with a second permeable region. The structures provide enhanced immunoisolation, which promotes the survival of even xenogeneic cells implanted in host tissue.

# FIG.3

## Description

## Field of the Invention

The invention generally relates to systems and methods for implanting materials into body tissue. In a more particular sense, the invention relates to the structures and methods for implanting living xenogeneic cells in host tissue within permeable membrane structures for achieving a desired therapeutic effect, such as, for example, the treatment of diabetes.

## Background of the Invention

Many diseases could be treated in a more physiologic fashion if tissue from lower animals could be transplanted into humans. However, the problem with discordant xenografting is hyper-acute rejection of the transplanted tissue.

Immunoisolation, as its name implies, is the protection of transplanted organs, tissues, cells, etc. from attack by the host immune system. Isolation from the host immune system is accomplished by the use of a semipermeable membrane. Therefore, utilizing immunoisolation, it is theoretically possible to perform both allografts and xenografts.

As early as 1949, Algire et al. showed that, even while allografts could be protected within a cell impermeable membrane, xenografts would be rapidly destroyed. More recently, other researchers have evaluated the use of AMICON XM-50® hollow fiber membrane for implanting xenogeneic tissue to treat diabetes. Results from these studies included a high number of failed devices generally characterized by fibrosis surrounding the implant. Recently, Lacy et al. has demonstrated diabetes correction in rodents using peritoneal implants of modified AMICON XM-50® membranes containing xenogeneic pancreatic islets. While fibrosis was minimized, the implant was not able to sustain high densities of viable islet tissue in rodents. Extrapolation of the results of Lacey et al. suggests that the size of the device needed to correct diabetes in humans is, practically speaking, too large, thereby limiting the clinical usefulness of this approach.

Recent work by Brauker et al. has demonstrated that a prescribed membrane architecture could promote vascular structures near the host tissue-membrane interface. Such membranes had pores that were formed by membrane structures (strands or fibers) with a pore diameter of less than 5µm, whereas membranes that did not develop near vascular structures had cavities with "plate-like" qualities, having pore diameters greater than 5 µm. Histological examination of the vascularizing membranes revealed that the invading inflammatory cells (of the host) had a rounded morphology, while the cells were flattened in the membranes that did not have close vascular structures. See, Brauker et al., Neovascularization of Synthetic Membranes Directed by Membrane Microarchitecture, J. Biomed. Mat. Res., In Press; Brauker, J., Martinson, L., Young, S., Johnson, R. C.: Neovascularization at a membrane-tissue interface is dependent on microarchitecture. Transactions of the Fourth World Biomaterials Congress April 24-28, 1992 p. 685; Brauker, J., Martinson, L., Carr-Brendel, V. E., and Johnson, R. C.: Neovascularization of a PTFE membrane for use as the outer layer of an immunoisolation device. Transactions of the Fourth World Biomaterials Congress April 24-28, 1992 p. 676; Brauker, J., Martinson, L., Hill, R., and Young, S.: Neovascularization of immunoisolation membranes: The effect of membrane architecture and encapsulated tissue. Transplantation 1:163,1992; and Brauker, J., Martinson, L. A., Hill, R. S., Young, S. K., Carr-Brendel, V. E., and Johnson, R. C.: Neovascularization of immunoisolation membranes: The -effect of membrane architecture and encapsulated tissue. Transplantation Proceedings 24:2924, 1992; copending Brauker et al. U.S. Patent Application Serial No. 08/210,068, filed March 17, 1994, entitled "Close Vascularization Implant Material."

Membranes of the type characterized by Brauker et al. facilitate high levels of vascularization near the membrane-host tissue interface. When used in association with an immunoisolation membrane, these membranes are capable of supporting allografts at high tissue densities for extended periods, even in the absence of immunosuppressive drugs.

Still, when implanted in such membranes, xenogeneic tissue does not survive.

## Summary of the Invention

One aspect of the invention provides a robust, permeable structure for implanting in host tissue. The permeable structure includes a first permeable region comprising a polymer solution formed-in-place in contact with a second permeable region. This aspect of the invention also provides methods for making devices for implanting in host tissue using the permeable structure, as well as methods for implanting living cells within such devices in host tissue.

In one embodiment, the permeable structure forms a chamber to hold living cells. The first permeable region surrounds at least a portion of the chamber and has a conformation that, when implanted in host tissue, substantially blocks penetration of host cells into the chamber while permitting solute transport. The second permeable region overlies the first permeable region and has a conformation that, when implanted in host tissue, forms a permeable interface with host tissue that permits solute transport. In this embodiment, the first permeable region comprises a solution of polymer material formed in place in contact with the second permeable region.

Additional characteristics for the confirmation of the first permeable region can vary. In a preferred embodiment, the conformation of the first permeable region, when implanted in host tissue, substantially blocks the penetration of at least host inflammatory cells into the chamber, thereby providing protection for allogeneic implants.

In a preferred method for manufacturing devices made from the permeable structure, the polymer material of the first permeable region is cast on the second permeable region and then coagulated in place to form the desired permeable conformation.

The inventors have observed that the death of xenogeneic tissue inside an immunoisolation device, otherwise capable of promoting near vascularization with allografts, is associated with massive cellular host response outside the device. This response, in turn, leads to a decrease in vascularization at the membrane-host tissue interface.

In the past, conventional wisdom has assumed that the destruction of xenografts can be prevented by providing membranes having pore sizes sufficiently small to prevent the ingress of host inflammatory cells and host immunogenic factors. The inventors have discovered that membranes that prevent the transport of these solutes from outside the device nevertheless do not prevent a very strong local inflammatory response in host tissue to implanted xenogeneic cells. The inventors believe that the local immune response is initiated by the escape of certain solutes from within the device. The solutes are shed by the xenogeneic cells themselves, and they appear to be lower molecular weight than IgG. Their blockage by the membrane therefore requires smaller pore size than previously recognized.

Still, xenogeneic cell viability also demands that the immunoisolation membrane possess a permeability sufficient to sustain the transport of nutrients to the implanted cells and the discharge of therapeutically important products and waste products from the implanted cells through the membrane. The inventors have discovered that the permeability of the membrane must strike a critical balance between transport of essential nutrients to the xenogeneic cells from nearby host vascular structures and the blockage of xenogeneic solutes from the cells that initiate local host immune response.

Another aspect of the invention thus provides immunoisolation membranes, devices, and methods that promote one survival of living xenogeneic cells implanted in host tissue. The membrane, devices, and methods provide a permeable boundary between the implanted xenogeneic cells and host tissue that has a prescribed permeability profile. The permeability profile reduces the initiation of a local immune response by antigens shed by xenogeneic cells within the boundary. The permeability profile also protects the xenogeneic cells from ingress of host inflammatory cells and host molecular immunogenic factors. Still, the permeability profile also provides transport of nutrients from the host sufficient to sustain xenogeneic cell viability.

In one embodiment, the invention provides a permeable structure forming a chamber to hold living cells. The structure includes a first permeable region surrounding at least a portion of the chamber, and a second permeable region overlying the first permeable region which, when implanted in host tissue, forms an interface with host tissue. The first permeable region comprises a solution of polymer material formed in place in contact with the second permeable region. The structure has a permeability for solutes of molecular size generally equal to or less than vitamin $B_{12}$ of at least $0.1 \times 10^{-4}$ cm/sec and a permeability for solutes of molecular size equal to or greater than IgG of less than $1.0 \times 10^{-7}$ cm/sec.

In a preferred embodiment, the structure has a permeability for solutes of molecular size generally equal to or less than vitamin $B_{12}$ of at least $0.5 \times 10^{-4}$ cm/sec and a permeability for solutes of molecular size equal to or greater than IgG of less than $1.0 \times 10^{-8}$ cm/sec.

Other features and advantages of the inventions will become apparent upon review of the following specification, drawings, and claims.

## Brief Description of the Drawings

Fig. 1 is a perspective view of an implant assembly that embodies features of the invention, being held in a clinician's hand;

Fig. 2 is an enlarged perspective view of the implant assembly shown in Fig. 1;

Fig. 3 is a side section view of the implant assembly taken generally along line 3-3- in Fig. 2;

Fig. 4 is a side section view of an alternative embodiment for the implant assembly;

Fig. 5 is a perspective view of a casting chamber containing apparatus used to manufacture cast membrane structures that embody features of the invention;

Figs. 6 to 11 show the steps in manufacturing the cast membrane structure using the apparatus shown in Fig. 5;

Fig. 12 is an exploded perspective view of a holder for containing samples of the cast membrane structure manufactured according to the invention, for the purpose of characterizing the permeability of the cast membrane structure according to another aspect of the invention; and

Figs. 13 and 14 show the diffusion cells and associated apparatus used to characterized the permeability of the cast membrane structure.

The invention may be embodied in several forms without departing from its spirit or essential characteristics. The scope of the invention is defined in the appended claims, rather than in the specific description preceding them. All embodiments that fall within the meaning and range of equivalency of the claims are therefore intended to be embraced by the claims.

### Description of the Preferred Embodiments

### I. The Implant Assembly

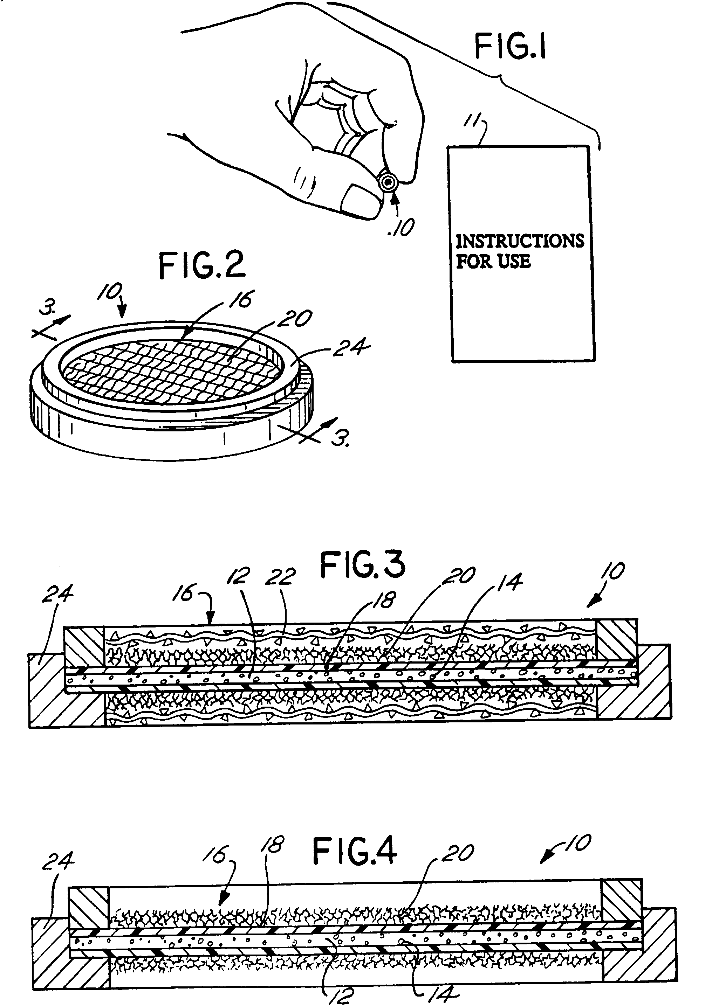

Figs. 1 to 4 show an implant assembly 10 that embodies the features of the invention.

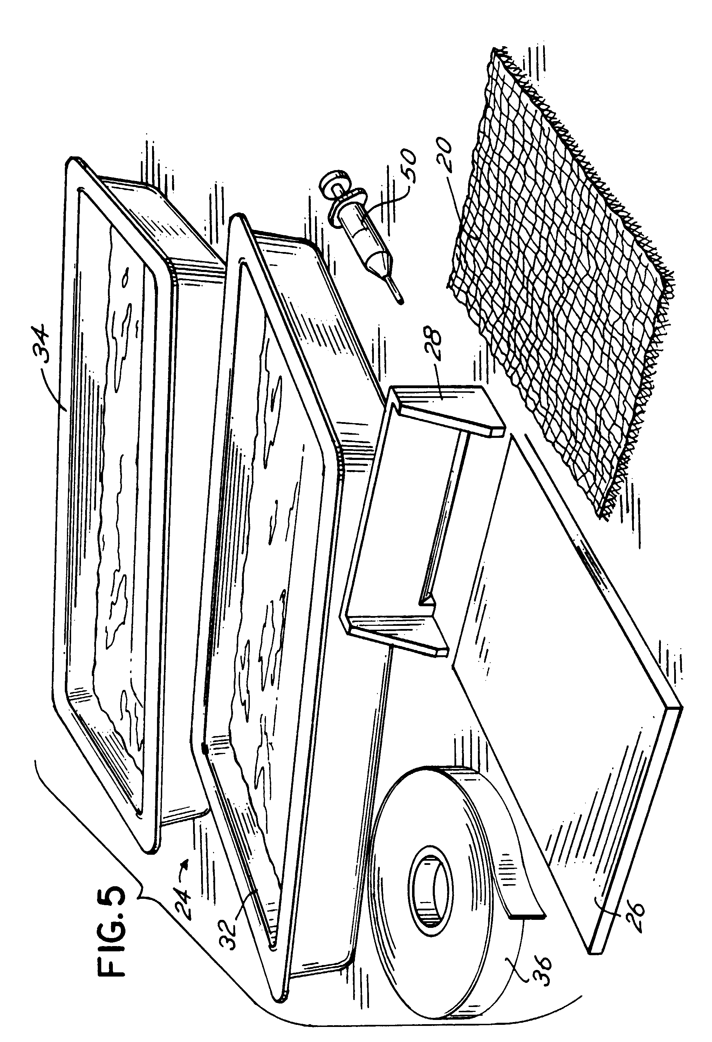

The assembly 10 forms a chamber 12 (see Fig. 3) to hold living cells 14 while implanted in host tissue. The implanted cells 14 generate biological products that the host, because of disease or injury, cannot produce for itself. For example, the chamber 12 can carry clusters of pancreatic cells (called "islets"), which generate insulin for release into and use by a diabetic host. In the illustrated and preferred embodiment (see Fig. 1), the assembly 10 also includes written instructions 11 that teach the enclosure of living cells within the chamber 12 and the implantation of the chamber 12 with enclosed living cells in host tissue. Exemplary procedures for preparing cells for implanting, for preparing the device for implanting, and for implanting the device with living cells in host tissue are set forth in, respectively, Sections IV(C) ; (D); and (E) of this Specification.

In the embodiment shown in Fig. 2, the assembly 10 is carried within a hoop-like housing 24. The details of construction of the hoop-like housing 24 are disclosed in U.S. Patent 5,344,454, which is incorporated herein by reference.

The assembly 10 forms a permeable, life-sustaining boundary 16 between the implanted cells 14 and the host. The permeable boundary 16 is characterized in terms of its ultimate physical strength and its permeability profile. The boundary 16 serves to isolate the implanted tissue cells from the immune response of the host. The boundary 16 also serves to transfer nutrients and waste products in support of the metabolic processes of implanted cells.

Regardless of whether the implanted cells 14 are xenogeneic, allogeneic, or isogeneic, the boundary 16 possesses an ultimate strength value that is sufficient to withstand, without rupture, the growth of new vascular structures, the growth of new cells within the chamber 12, and other physiological stresses close to the host tissue. Keeping the boundary 16 secure assures isolation of the implanted cells from both the immunogenic factors and inflammatory cells of the host.

These physiological stresses are caused when the host moves about in carrying out its normal life functions. The proliferation of implanted cells and the growth of vascular structures also contributes to the physiological stresses close to the boundary 16. The stresses challenge the physical integrity of the boundary 16 by stretching or otherwise deforming it. Absence of sufficient ultimate strength value and normal physiological stresses can rupture the boundary 16, exposing the implanted cells to the full effect of the host's immune and inflammatory systems.

The inventors presently believe that ultimate strength values sufficient to withstand physiological stresses close to the host tissue without rupture in animals lie above about 100 pounds per square inch (PSI). The ultimate strength values are determined by measuring the tensile strength of the material. Tensile strength is measured by ASTM D-412.

Also regardless of the type of implanted cell, the boundary 16 must possess a permeability profile that sustains a flux of nutrients into the chamber 12 and waste products from the chamber 12 sufficient to sustain the viability of the implanted cells.

In the illustrated and preferred embodiment, the permeable boundary comprises a first permeable region 18 and a second permeable region 20. According to one aspect of the invention, the first permeable region 18 is formed-in-place in contact with the second permeable region 20, created a robust, integrated boundary 16 possessing the requisite ultimate strength. Furthermore, the parameters for manufacturing the formed-in-place integrated, two-region boundary 16 can also controlled to provide the permeability profile required to sustain xenografts, allografts, and isografts.

### A. The First Permeable Region

The first permeable region 18 immediately surrounds the chamber 12 containing the implanted cells 14. It is this region 18 that is formed-in-place. The first region 18 possesses multiple characteristics.

Regardless of the type of implanted cell 14 (i.e., xenogeneic, allogeneic, or isogeneic), the first region 18 has a pore size sufficient to block penetration into the chamber 12 by host cells. This penetration breaches the integrity of the boundary 16, exposing the implanted cells 14 to the complete immune response of the host. Generally speaking, pore sizes less than about 2 μm (i.e., 2 micrometers) will block the ingress of host cells. As used in this Specification, "pore size" refers to the maximum pore size of the material. For pore sizes greater than about 0.1 microns, the practitioner determines pore sizes using conventional bubble point methodology, as described in Pharmaceutical Technology, May 1983, pages 36 to 42. For pore sizes smaller than this value, the practitioner usually relies upon transport characterization to infer the size of passageways in the material. In this Specification, the term "pore" will generally refer to transport passageways of all sizes.

### (I) Xenografts

When the implanted cells 14 are from another animal species (i.e., xenografts), the pore size of the first region 18 must also be sufficient to prevent the passage of both inflammatory cells and molecular immunogenic factors from the host into the implant tissue chamber. As used in this Specification, "inflammatory cells" include macrophages, foreign body giant cells, and fibroblasts, and "molecular immunogenic factors" refers to molecules such as antibodies and complement. Pore sizes sufficient to block passage of both inflammatory cells and molecular immunogenic factors in humans lie in the range of about .015 micrometers (also referred to as "microns" or as *μm"). Of course, these pore sizes are also impermeable to vascular structures.

Furthermore, with xenografts, the first permeable region 18 also must block the passage through the boundary 16 of macromolecular antigens that are shed by the xenogeneic cells contained within the chamber 12. The exact molecular size of these macromolecular antigens is not known, but they are believed to be smaller than IgG.

Of particular interest in this regard, and as will be described in greater detail later, the formed-in-place first region 18 can be manufactured from natural or synthetic biocompatible polymers which present a permeability that supports xenogeneic cell metabolism in vivo, and which also demonstrates in vivo the ability to restricts the passage of shed xenogeneic antigens, thereby reducing or preventing the host immune response to the xenogeneic cells.

### (ii) Allografts

When the implanted cells are from the same animal species but have a different genetic make up (i.e., allografts), the pore size of the first region 18 usually must be sufficient to prevent the passage of only inflammatory cells from the host into the implant cell chamber. In allografts, molecular immunogenic factors do not seem to adversely affect the viability of the implanted cells. Still, some degree of tissue matching may be required for complete protection. Pore sizes sufficient to block passage of inflammatory cells in humans lie in the range of below about 0.6 micrometers. These pore sizes, too, are impermeable to vascular structures.

### (iii) Isografts

When the implanted cells are isografts (autologous implants or genetically engineered cells), the pore size must be sufficient only to prevent the isografts from exiting the chamber 12, while also preventing ingress of host cells into the chamber 12.

### B. The second Permeable Region

The second permeable region 20 of the boundary overlies at least a portion of the first region 18, which is formed-in-place upon it. The second region 20 constitutes the interface with host tissue.

In the illustrated and preferred embodiment, the second region 20 has an architecture that promotes the growth of vascular structures in host tissue near the interface. Preferably, at least some of the near vascular structures lie within one cell thickness of the interface.

It is through the second region 20 that the permeable boundary 16 associates itself with the host's biological system closely enough to transfer nutrients and wastes in support of the biological processes of the implanted cells 14. The permeable boundary 16 also transfers the therapeutic products generated by the implanted cells 14 to the host.

Vascularization near the host tissue-boundary interface occurs if the three dimensional conformation of second region 20 promotes certain host inflammatory cell behavior. Brauker et al. have demonstrated that membranes that did have near vascular structures allowed cellular penetration and had pores that were formed by membrane structures (strands or fibers) with a pore diameter of less than 5μm, whereas membranes that did not develop close vascular structures had cavities with "plate-like" qualities, having pore diameters greater than 5μm. Histological examination of the vascularizing membranes revealed that the invading cells had a rounded morphology, while the cells were flattened in the membranes that did not have close vascular structures. The cells appear to be "trapped" and not allowed to flatten on any surface, which apparently causes the more rounded morphology of the cells which infiltrate the vascularizing membranes. The hypothesis is that the membrane architecture dictates cellular morphology, and the rounded cells in turn secrete some, as yet unknown, trophic factors which promote the formation of vascular structures.

Accordingly, the material for the second region 20 is a polymer membrane having an average nominal pore size of approximately 0.6 to about 20 μm, using conventional methods for determination of pore size in the trade. Preferably, at least approximately 50% of the pores of the membrane have an average size of approximately 0.6 to about 20 μm.

The structural elements which provide the three dimensional conformation may include fibers, strands, globules, cones or rods of amorphous or uniform geometry which are smooth or rough. These elements, referred to generally as "strands," have in general one dimension larger than the other two and the smaller dimensions do not exceed five microns.

In one arrangement, the material consists of strands that define "apertures" formed by a frame of the interconnected strands. The apertures have an average size of no more than about 20 μm in any but the longest dimension. The apertures of the material form a framework of interconnected apertures, defining "cavities" that are no greater than an average of about 20 μm in any but the longest dimension.

In this arrangement, the material for the second region 20 has at least some apertures having a sufficient size to allow at least some vascular structures to be created within the cavities. At least some of these apertures, while allowing vascular structures to form within the cavities, prevent connective tissue from forming therein because of size restrictions.

Further details of the material are set forth in copending U.S. Application Serial No. 08/210,068 entitled "Close Vascularization Implant Material" filed March 17, 1994, which is incorporated into this Specification by reference.

In a preferred implementation, the second region 20 comprises a membrane material made by W. L. Gore and Associates (Elkton, Maryland) under the trade name Gore-Tex™ The Gore-Tex™ material comprises a microporous membrane made from poly(tetrafluoroethylene) (PTFE). The membrane is 15 microns thick and has a pore size of 5 microns. Polyester strands 22 (see Fig. 3) normally join the PTFE membrane to form a backing for it. The backing 22 has a depth of about 120 microns. However, when the first and second regions 18 and 20 are formed-in-place together according to the invention, the polyester strand backing 22 need not be present and made be removed (as Fig. 4 shows).

In another embodiment (not shown), the second region 20 comprises a permeable membrane structure formed with multiple microfabricated layers of polymer film made, for example, from photoimageable polyimide material. The film is processed, using either negative photoresist techniques or etchable membrane fabrication techniques, to create predefined geometric patterns of holes and intermediate spaces defining strands. The geometric patterns alternate between film layers from smaller, more closely spaced hole patterns (with cross hole dimensions equal to or less than about 20 μm and strand dimensions typically about 2-3 μm) to larger holes with less closely spaced patterns (with cross hole dimensions exceeding 20μm and upwards to about 50μm and strand dimensions typically about 4-5μm). The stacking of different geometric patterns creates an open, permeable membrane structure having randomly positioned, interconnected cavities with minimum interior dimensions greater than about 5 μm formed by interconnected stands with minimum dimensions less than about 5 μm.

Further details of this construction are described in copending U.S. Application Serial No. 08/320,199 entitled "Porous Microfabricated Polymer Membrane Structure" filed October 7, 1994, which is incorporated into this Specification by reference.

The preferred embodiment shows the permeable membrane structure being used in association with a chamber for implanting living cells. Still, it should be appreciated that the permeable structure embodying the features of the invention can be used in association with other implanted objects, like catheters, biosensing devices, and the like.

## II. Manufacture of the Permeable Boundary

The permeable boundary 16 functions as a synthetic membrane between host tissue and the cells 14 contained in the chamber 12. With a concentration gradient as the driving force, the boundary membrane permits the selective transport of certain molecular solutes between host tissue and the cells 14, while blocking the transport of other molecular solutes. The nature of the transport and blockage functions depends on prescribed, discriminating interactions between the physical configuration of the membrane and the physical configuration of the transported material. For synthetic membranes these interactions are based for the most part on the size of the transported material relative to the size and distribution of the passageways or pores within the membrane. In synthetic membranes, the size of the membrane pores often varies, sometimes over a broad range. In addition, the diffusion process within the various membrane pores can be significantly hindered relative to that in free solution by interactions of the solute with the pore walls, even when the solute is several times smaller than the pore.

The criteria in developing an immunoisolating membrane includes the creation of a structure for which (i) the largest pore will prohibit passage of a defined type or types of solute, and (ii) the distribution of pore size has a mean sufficiently close to the maximum pore size so that the diffusion of the desired small solutes is not overly hindered by a large population of very small pores.

The first permeable region 18 is made by a process of controlled phase change, called "casting," in which the membrane material, a high molecular weight synthetic polymer, is first dissolved in a solvent, then brought into contact with the second permeable region 20, and then exposed to conditions which cause the material to precipitate from solution in a macroscopically continuous phase adjacent to the second region 20. Careful selection and control of membrane material, solvent system, concentration, and phase inversion conditions determine the final membrane micro-architecture.

The polymer material for the first region 18 can comprise a natural or synthetic polymer selected based upon considerations that include its biocompatibility, its ease of fabrication, its ability to consistently form predictable, identifiable pore geometries and distributions, its chemical inertness, and its strong mechanical properties. This Specification specifically identifies five polymers that meet the above criteria; namely, cellulose acetate (CA); poly(ethylene-co-vinyl alcohol) (EVOH) ; cuprammonium cellulose (CAC); poly(acrylonitrile) (PAN); and various acrylic copolymers, such as used in commercial yarns. However, it should be appreciated that other natural and synthetic polymers meet these criteria, such as hydrophillic polyurethanes; poly(vinyl alcohol); and cellulose dissolved by various processes.

**A. Casting the Polymer Dope**

Figs. 5 to 11 show the steps in the casting process performed in accordance with the invention. These steps are performed, regardless of the particular polymer selected.

The membrane casting process is preferably carried out in an enclosed casting chamber 24 (see Fig. 5) within a standard laboratory fume hood at ambient room temperature. The casting chamber 24 permits the continuous flow of filtered air over the casting area.

Present within the casting chamber 24 (see Fig. 5) are a casting plate 26, a casting blade 28, a syringe 30 containing dissolved polymer solution or dope, and various processing baths, two of which Fig. 5 shows. One bath 32 comprises a cooled, thermostated coagulation bath. Another bath 34 comprises an extraction bath. Other baths (not shown) comprise various aqueous baths.

The casting plate 26 is preferably polished stainless steel, but it can also be mirror glass. Its dimensions can vary. In one representative embodiment, the casting plate 26 is stainless steel and measures 6.25 inch by 7.25 inch, with a thickness of about 0.25 inch.

The plate 26 is cleaned, either with Contrad 70 (Curtin-Matheson Scientific) for steel or Chromerge (Mallinckrodt) for glass, rinsed thoroughly, dried in open air, flushed with filtered air, and then placed immediately into the casting chamber 24.

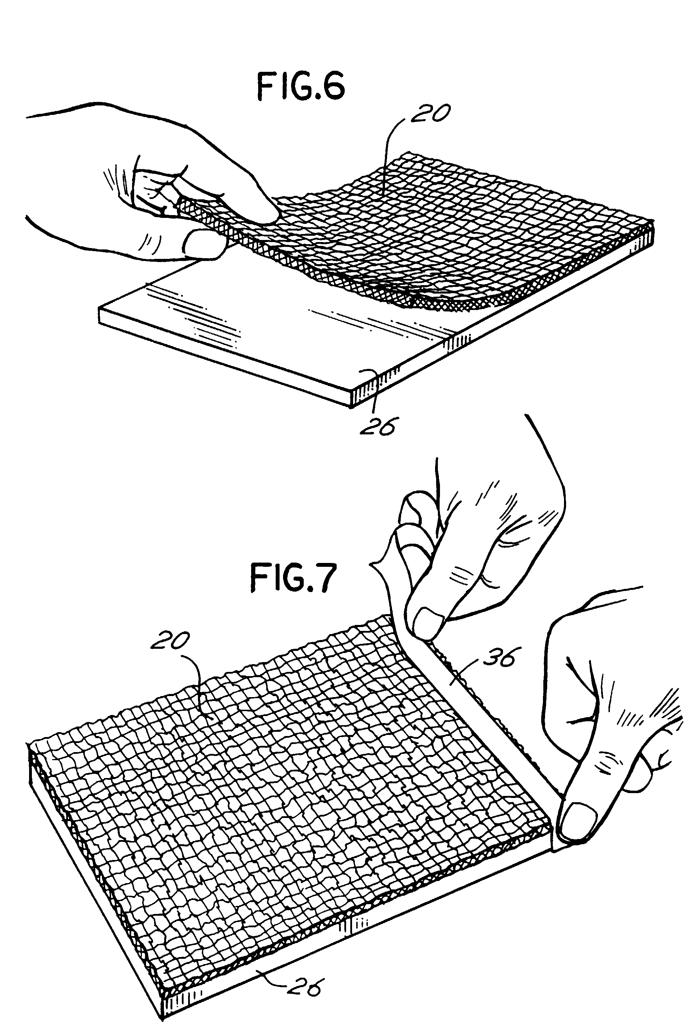

Referring now to Figs. 6 and 7, the second permeable region 20 (in the form of the GORE-TEX material) is peripherally secured to the casting plate 26 within the casting chamber 24, using tape 36, adhesive, or both. The surface of the region 20 which, when implanted, faces host tissue lies face down on the casting plate 26. It is important that the second region 20 lays in intimate surface contact, free of wrinkles, against the entire casting plate 26. The casting plate 26 may be made with a slightly convex surface to assure intimate, wrinkle-free surface contact, particularly between the midportion of the plate 26 and the second region 20.

The syringe 30 contains the polymer material selected for the first region 18, which is dissolved in dope form in a selected solvent. The details of the dissolution process will vary according to the polymer used, and these details will be described later. After dissolution, the polymer dope is drawn up into the syringe 30 (which can be a 5 cc polypropylene syringe manufactured by Becton-Dickinson). The syringe 30 is capped, flushed with filtered air, and quickly placed in the casting chamber 24.

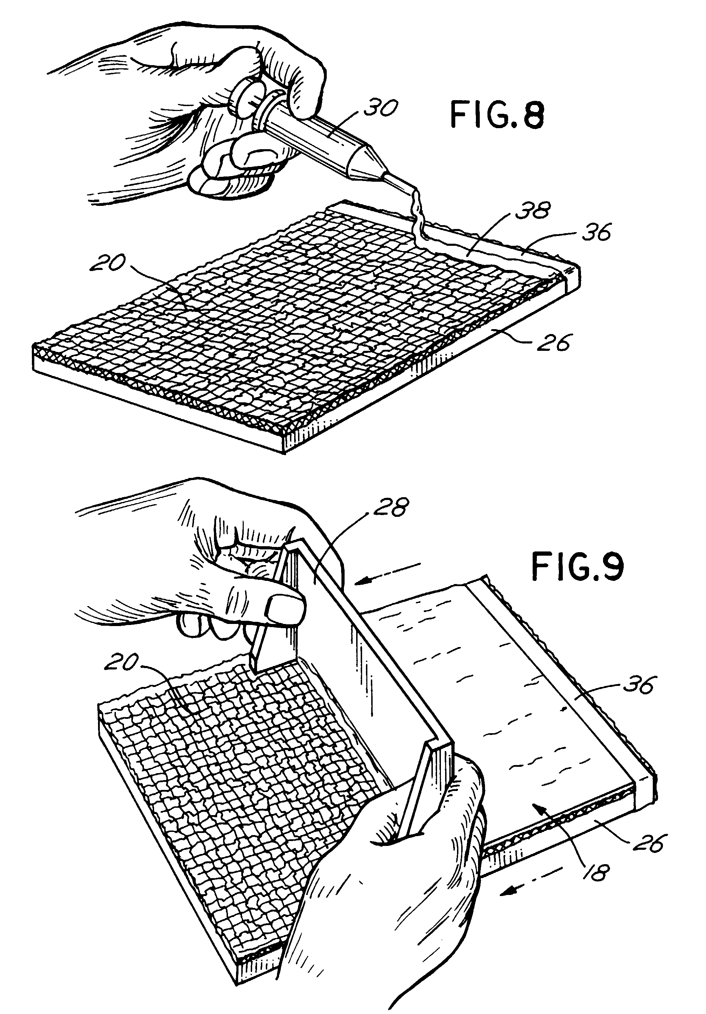

As Fig. 8 shows, within the casting chamber 24, the polymer dope is manually dispensed from the syringe 30 in an elongated bead 38 (typically about ⅛-inch in diameter) along the upper portion of the second region 20 on the casting plate 26. As Fig. 9 shows, the dope bead 38 is then spread out or "drawn down" over the surface of the second region 20 by slowly dragging the casting blade 28 along the length of the plate 26. The thickness of the dope drawn down on the second region 20 depends upon the size of the gap that separates the edge of the casting blade 28 from the exposed surface of the second region 20. The size of the gap can vary between 18 μm to about 107 μm depending upon the polymer used and the thickness desired.

It is desirable that a portion of the polymer dope actually penetrates the surface of the second region 20 to some extent. This penetration assures that a secure, physical bond develops to hold the second region 20 and cast first region 18 together.

Depending upon the surface tension characteristics of the second region 20 and the cast polymer used, it may be necessary to pretreat the second region 20 with a water soluble surfactant like FC-135, Pluronic, or Triton X100, before applying the dope bead. The pretreatment controls the adhesion and depth of penetration of the drawn-down dope.

For example, pretreating the PTFE surface of a GORE-TEX material with a 0,1% FC-135 solution is recommended before casting with a CA polymer. Likewise, pretreating the PTFE surface of a GORE-TEX material with a 0.2% FC-135 solution is recommended before casting with an acrylic polymer. No pretreatment of the PTFE surface with surfactant is required before casting with an EVOH polymer.

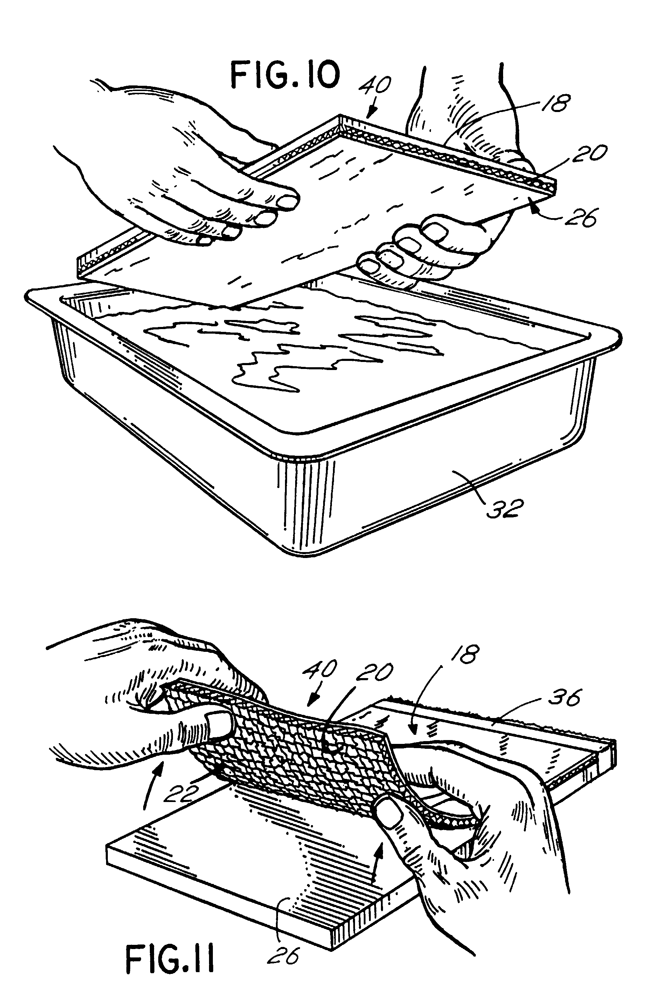

As Fig. 10 shows, once the polymer dope has been drawn down along nearly the entire second region 20, forming a membrane-cast structure 18, the casting plate 26 is lifted and slid smoothly into the pre-conditioned coagulation bath 32. The contents of the coagulation bath 32 will vary according to the polymer used, and detailed examples will be described later. The desired permeable structure of the first region 18 forms in the coagulation bath.

The presence of the second region 20 between the cast membrane structure 18 and the casting plate 26 provides a near zero temperature gradient across the membrane cast structure 18 within the cooled, thermostated coagulation bath 32. This, in turn, provides greater control over the formation of the permeable structure desired for the first region 18. The second region 20 serves as an insulator, so that the cast first region 18 does not directly contact the casting plate 26 and experience the often large temperature difference between it (at a cooled temperature) and the casting plate 26 (at ambient temperature) when placed the coagulation bath 32. The presence of the second region 20 mediates the temperature difference, leading to more uniform and predicable pore formation in the first region 18 and adhesion between the first and second regions 18 and 20.

After a prescribed period of time (usually about 5 minutes), the plate 26 and membrane-cast structure 40 are removed as a unit from the coagulation bath 32 and placed in a pre-conditioned extraction bath 34. The contents of the extraction bath 34 will vary according to the polymer used, and detailed examples will be described later.

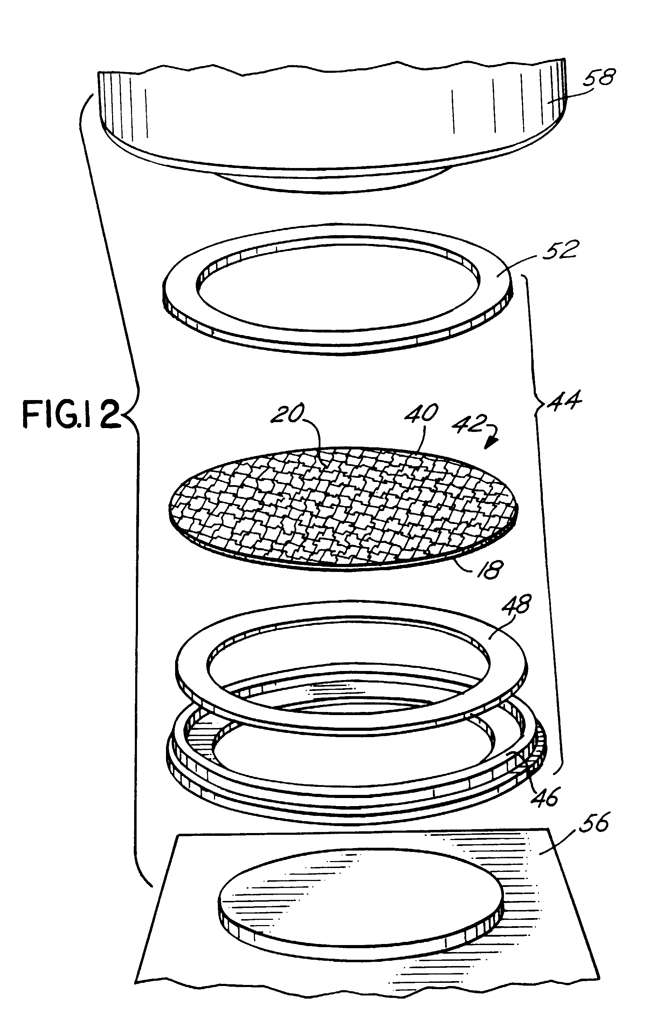

After a prescribed period of time in the extraction bath 34 (again, usually about 5 minutes), the cast-membrane structure 40 is gently removed from the casting plate 26, as Fig. 11 shows. It is then allowed to float freely, cast side down, in a series of water baths (not shown).

The cast-membrane structure 40 is plasticized before drying to prevent collapse of the pore structure. This is accomplished by soaking the cast-membrane structure 40 in glycerol solutions of various concentrations. The plasticized cast-membrane structure 40 is carefully laid out onto polypropylene supports, secured peripherally with masking tape, and allowed to dry, at least overnight before testing or use.

As before described, once the first region 18 has been formed-in-place upon the second region 20, the polyester backing material 22 of the GORE-TEX material can be peeled off, as Fig. 4 illustrates. The formed-in-place first region 18 provides sufficient support for the PTFE second region 20, without the need for additional backing.

In an alternative process (not shown), the dope bead 38 can be applied and drawn down directly on the casting plate 26 using the casting blade 28 , without the second region 20 being present. Once the dope is drawn down, the second region 20 (pretreated with surfactant, if required) is placed PTFE side down upon the casting plate 26 in intimate contact with the dope polymer. The drawn down dope polymer is allowed to wick partially into the second region 20, which is also then peripherally secured to the casting plate 26. The cast-membrane structure 40 is then placed in the coagulation bath 32, as previously described, followed by the subsequent extraction and water baths 34, plasticization, and drying, also as previously described.

The membranes prepared from the polymer solutions, as above described, can be broadly classified as hydrogels in the sense that, in the presence of water, they form highly solvated, three dimensional, permeable networks with local regions of close molecular association which provide strength to the network.

## B . Representative Polymer Dopes

### (I) Cellulose Acetate (CA)

Cellulose acetate 398-30 in powder form from Eastman Chemical can be used as received from the manufacturer. According to manufacturer's specifications, the material has a number average molecular weight, $M_n$, of 50,000 daltons and an acetyl content of 39.7%, corresponding to a degree of substitution of 2.44 moles of acetyl per mole of anhydroglucose unit. The powder is weighed out into wide mouth jars and a sufficient amount of N-methyl pyrrolidone (NMP, Baxter Burdick and Jackson) is added to give the desired final concentration. In casting boundaries for xenografts, concentrations of from 15 wt% to 20 wt % are recommended. The jar is placed on a roller mill and the contents gently mixed until dissolved (approximately 24 hours). The solution is filtered under pressure using a Gelman 4280 filtration funnel with 5 or 10 μm nylon membranes (MSI N50- and N99SP04700) prior to use. Solution viscosity at 25.0 °C is determined using a Brookfield DV-I viscometer (spindle #4) calibrated with viscosity standards of appropriate range (Brookfield). Castings of 15 wt% to 20 wt% are made, as described above, using a 72 μm blade gap at ambient temperature. The cast-membrane structure 40 is precipitated directly in the coagulation bath of deionized water at 2 °C. After 5 minutes, the cast-membrane structure 40 is removed from the coagulation bath and extracted sequentially in two room temperature water baths (4 L) for 15 minutes each. The cast-membrane structure 40 is then soaked in a 40 % glycerol solution for 30 minutes and air dried.

### (ii) Cuprammonium Cellulose (CAC)

Cuprammonium solutions of cellulose derived from cotton linters (Buckeye Cellulose) or wood pulp (ITT Rayonier, Inc.), or mixtures thereof are prepared in a small scale, high viscosity laboratory reactor. The reactor is first charged with tribasic copper sulfate, prepared separately by the stoichiometric addition of ammonium hydroxide to aqueous copper sulfate. Ammonium hydroxide (28 % solution) is then added to form equimolar amounts of tetra amine copper sulfate and tetra amine copper hydroxide, the actual solvating agent for cellulose. This mixture is then cooled to about 6 °C and previously washed cellulose slowly added with stirring to form a thick semi-dissolved slurry. Subsequent addition of sodium hydroxide solution converts the remaining tetraaminecopper sulfate to the hydroxide thereby dissolving the remaining cellulose. This solution (initially about 9 wt % cellulose) is finally diluted with ammonium hydroxide solution to give a working concentration of 7 wt %. Viscosity is determined as described above. Because of the sensitivity of the cuprammonum solution to oxidative degradation, all solutions are stored under nitrogen.

Cast-membrane structures 40 are cast as described above using a 107 µm blade gap, allowed to stand for a predetermined evaporation time (typically up to five minutes), coagulated in 2 w/v % sodium hydroxide for 0.5 minutes, rinsed with water, de-coppered in 5 w/v % sulfuric acid until clear, rinsed again, plasticized in 10 v/v % glycerol for 30 minutes, and dried. The permeability profile of the resulting membrane can be precisely controlled by adjusting the evaporation time.

### (iii) Poly (ethylene-co-vinyl alcohol) (EVOH)

EVOH is available in various molecular weights and monomer ratios from EVAL Company of America. The E-series material, containing 44 mole % ethylene, is used. The E-series material dissolves at elevated temperature in both NMP and dimethylsulfoxide (DMSO) and remains in solution on cooling. Solutions were prepared by preheating either NMP or DMSO in a round bottom flask equipped with a crescent-type stirrer and slowly adding the polymer pellets. After complete dissolution, which typically took 4 to 6 hours at 80 °C, the solution was cooled and pressure filtered as described above for the CA casting solutions. Brookfield viscosities were determined at 25 °C using spindle #4. The cast-membrane structures 40 were prepared in the standard manner described above, using a 36 µm blade gap. The cast-membrane structures 40 were coagulated in a 40 v/v % glycerol solution for 30 minutes. The cast-membrane structures 40 were plasticized in a solution of the same glycerol concentration and dried as previously described.

Compared to the other polymers described herein, when prepared as described above, the EVOH membrane appears to have enhanced bond strength to the permeable support, as evidence by the relative difficulty in pulling the formed structured apart.

### (iv) Poly(Acrylonitrile) (PAN)

PAN in powder form from Polysciences can be used as received from the manufacturer. The powder is weighed out into wide mouth jars and a sufficient amount of DMSO is added to give the desired final concentration. In casting boundaries for xenografts, concentrations of about 15 wt% PAN are recommended. The remainder of the dissolution and casting process is as described for CA cast-membrane structures 40 above.

### (v) Acrylic Copolymers

ACRILAN yarn purchased from (Monsanto) can be used as received from the manufacturer. The yarn is weighed out into wide mouth jars and a sufficient amount of DMSO is added to give the desired final concentration. In casting boundaries for xenografts, concentrations of about 15 wt% acrylic are recommended. The remainder of the dissolution and casting process is as described for CA cast-membrane structures 40 above.

### III. Characterizing Permeable Boundary Permeability

The permeability of the cast-membrane structures 40 as described above can be characterized using a stirred diffusion cell and multi-solute test methodology, which will be described now. The described diffusion cell and multi-solute test methodology provide the accurate and precise determination of membrane permeability required to properly assess the suitability of a given membrane structure to serve in an immunoisolation device.

### A. Measuring Solute Transport Over Time

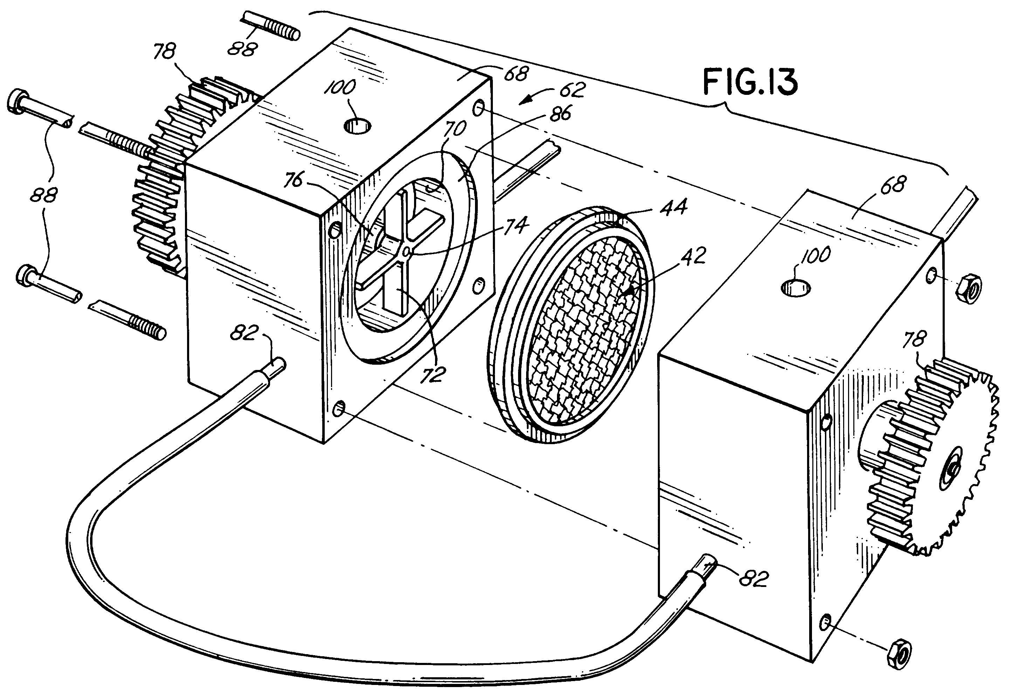

As Figs. 12 and 13 show, circular samples 42, 0.98" in diameter, are punched from a dried cast-membrane structure 40. The samples 42 are soaked in phosphate-buffered saline (PBS) and mounted in the membrane holders 44 shown in Fig. 13. As Fig. 12 best shows, the holders 44 use a stacking arrangement consisting of a housing 46; a silicone rubber gasket 48, 0.004"; the membrane sample 42; and an insert 54.

The silicone rubber gasket 48 is placed onto the inner ledge of the housing 46 which rests on the platform 56 of a small arbor press. The membrane sample 42 is then laid across the gasket 48. The slightly over-sized rigid insert 52 is pressed by means of the ram 58 into the housing flush with the top surface, compressing the gasket and membrane assembly to a predetermined level defined by the thickness of the various components.

In practice, the exact arrangement of gaskets/spacers and the membrane sample 42 may vary depending on the membrane thickness and mechanical properties and the amount of compression desired. The holder 44 decouples the sealing of the membrane samples 42 from the assembly of the diffusion cell and provides a convenient means of handling thin, delicate membranes.

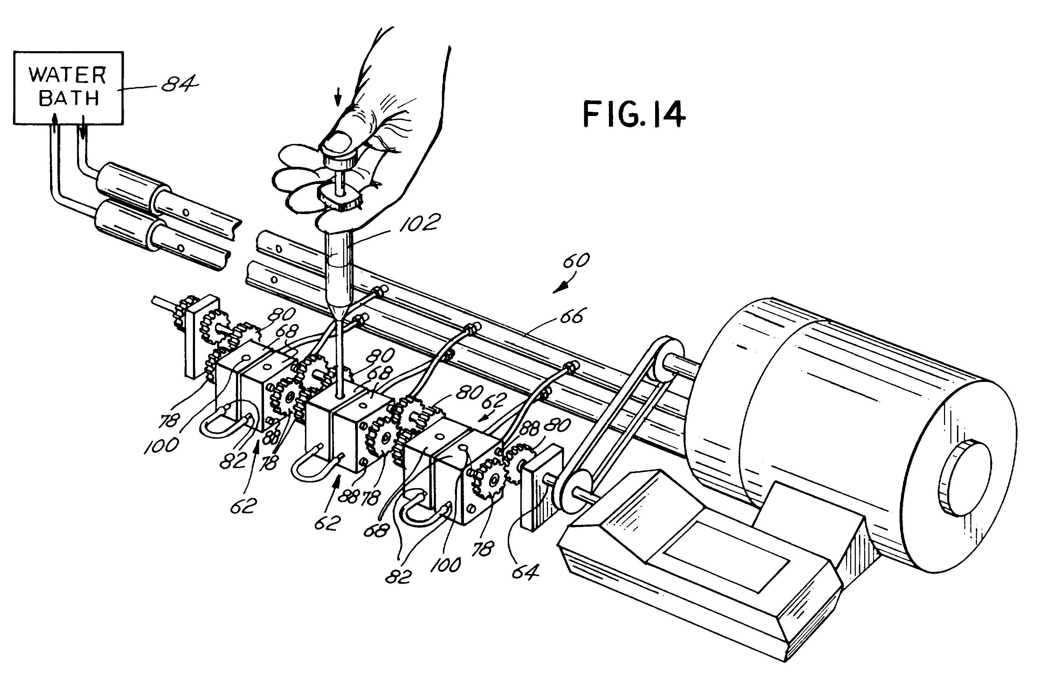

The diffusion apparatus 60 (see Figs. 13 and 14) consists of a number of identical diffusion cells 62 each connected to a common drive shaft 64 and constant temperature water manifold 66.

A single diffusion cell 62 is best shown in Fig. 13. The cell 62 consists of two 3.5 cm$^3$ cylindrical chambers separated by the membrane sample 42 which is fixed in the pre-assembled holder 44. Each cell half 62 consists of an acrylic block, 1.4" by 1.4" by 0.9", into which is machined a cylindrical pocket 70, which is 0.770" in diameter ($R_e$ - 0.978 cm) defining a transport area of 3.00 cm$^2$. A centrally mounted four blade impeller 72, which is 0.710" in diameter ($R_e$ - 0.902 cm) and 0.137" wide by 0.050" thick, is connected to a stainless steel shaft 74, which is 0.156" in diameter, mounted in an acetal bearing 76. The bearing 76 comprises a separately machined part, which is pressed into the body of the cell 68 along with two o-rings (not shown), providing an external static seal between the bearing 76 and the cell body 68 and an internal dynamic seal between the bearing 76 and the rotating shaft 74.

The shaft 74 extends through the distal end of the cell body 68 and is attached to an acetal spur gear 78, which mates with an identical gear 80 on the parallel drive shaft 64 (see Fig. 14). The lower portion of each cell half is pierced by a thin-walled stainless steel tube 82, which passes under the bearing 76 just behind the impeller 72, serving as a heat exchanger. Water at 37° C is caused to continuously flow through the steel tube 82 in each chamber from a water bath 84 via the manifold 66 during the permeability test period. As Fig. 14 shows, the tubes 82 from each mating cell half 68 are connected together by flexible tubing so that thermostated water flows serially from the common manifold 66, through each cell half 68, and back to the water bath 84.

As Fig. 13 shows, the face of each cell half 68 has a machined recess 86, 0.05" deep by 1.250" in diameter, which receives the membrane holder 44 and peripheral o-rings. The cell halves 68 are held together by two to four #4-40 screws 88.

Each cell half 68 includes an access port 100, which opens into the associated chamber. A mechanical pipette 102 (see Fig. 14) inserted through the access port 100 fills each chamber with a known volume of a physiological solution, such as PBS.

At the outset of the permeability test cycle (t = 0), the technician removes 100 µL of PBS from the left cell half 68 of each diffusion cell, using a mechanical pipette 102 inserted through the access port 100. The technician replaces this removed volume with 100 µL of a prescribed solute "cocktail" solution.

The solution contains:

 Tryptophan (Sigma T-0254) (0.5 mg/ml)
   Molecular Weight 204.2 D
 Vitamin B$_{12}$ (Sigma V-2876) (1.5 mg/ml)
   Molecular Weight 1355 D
 Myoglobin (Sigma M-0630) (6 mg/ml)
   Molecular Weight 17,000 D
 Albumin (Sigma A-8022) (22 mg/ml)
   Molecular Weight 65,000 D
 IgG (Sigma I-5506) (25 mg/ml)
   Molecular Weight 160,000 D

The solution is prepared by weighing out the appropriate amount of each solute, adding 10 mM PBS, gently mixing until dissolved, and finally filtering through a 0.22 µm syringe filter just prior to use.

With the addition of the solute cocktail at t = 0, the test cycle begins. Due to the presence of the solute cocktail in the left cell half, each diffusion cell has a high concentration (left) cell half and a low concentration (right) cell half. Over time, the test membrane will transport solutes in the direction of the concentration gradient from the left cell half chamber to the right cell half chamber. The amount of transport over time will depend upon the permeability of the test membrane to each of the solutes in the cocktail.

The permeability test cycle lasts 24 hours. At prescribed intervals during the test cycle, the technician simultaneously removes from each cell half 100 µL of solution, replacing the removed solution with 100 µL of fresh, preheated PBS. The removed solution for each cell at each interval is placed in sample vials, which are marked to identify each sample in terms of its cell number, cell half, and sample interval.

In the preferred implementation, the prescribed intervals for taking these samples are at t - 30 minutes; t - 1 hour; t - 2 hours; t - 5 hours; and t - 24 hours. At the end of the test cycle, 10 samples for each diffusion cell will have been taken.

For each sample, the concentration of each solute in the solute cocktail solution is measured using conventional high performance size exclusion chromatography (HPSEC) techniques. For each test interval, a paired chromatograph is developed, showing the concentrations for each solute on the high concentration (left) cell half and the low concentration (right) cell half. The change of the paired chromatographs over time are indicative of the permeability of the sample membrane for each of the solutes in the cocktail. For each test interval, the technician measures and records from the chromatographs the peak height for each solute in the high and low cell half.

**B. Deriving True Membrane Permeability**

The technician next quantifies the measured results, according to the following methodology, to derive true membrane permeability.

**(i) Determining Overall Mass Transfer Coefficients $k_o$**

The overall mass transfer coefficients ($k_o$) are determined based upon the following relationship:

$$\ln\left[1 - \frac{\beta C_2(t_i)}{F^{i-1}C_{1,avg}(t_o)}\right] = -\frac{2k_oA}{V_H} \times t_i \tag{1}$$

where:

the left side logarithmic function of Equation (1) represents the natural logrithm of the dimensionless concentration difference between the two chambers, and:

$\beta = 1 + V_2/V_1$

$F = 1 - V_S/V_H$

$V_1$ is the left chamber volume.

$V_2$ is the right chamber volume.

$V_H$ is the harmonic mean volume defined by $2/V_H - 1/V_1 + 1/V_2$.

$V_S$ is the sampling volume.

A is the area of the membrane sample.

$C_2$ is the solute concentration measured in the right chamber

$t_i$ is the time at the i-th sampling point.

$C_{1,avg}(t_o)$ is the initial concentration in the left chamber immediately after the addition of solute. This quantity can be obtained from a knowledge of the stock solution concentration. However, for more consistent results, this quantity should be estimated from the concentrations in both chambers at each of the N sampling times. The requirement for mass balance leads to:

$$\bar{C}_{1,avg}(t_o) = \frac{1}{NV_1}\sum_{i=1}^{N}\left\{C_1(t_i)V_1 + C_2(t_i)V_2 + V_s\sum_{j=1}^{i-1}[C_1(t_j) + C_2(t_j)]\right\}$$

$$\textbf{(2)}$$

The values for the left side logarithmic function of Equation (1) are plotted as a function of time for each solute. The least squares techniques is used to determine the best fit slope of the plotted function. Knowing the best fit slope, the technician solves the right side of Equation (1) for $k_o$.

**(ii) Determining Boundary Layer Mass Transfer Coefficient $k_b$**

The actual membrane mass transfer coefficient or permeability $k_m$ can be derived from the overall mass transfer coefficient $k_o$, since they are reciprocally related as follows:

$$\frac{1}{k_m} = \frac{1}{k_o} - \frac{2}{k_b} \tag{3}$$

where:

$k_b$ is the boundary layer coefficient for each side of the sample membrane, which is assumed to be the same for each side of the membrane.

The boundary layer coefficient $k_b$ takes into account that, even with vigorous stirring within the diffusion chamber,

11

there will always be a thin region on each side of the membrane which is not stirred as well as the rest of the fluid within the diffusion chamber. This creates concentration boundary layers that act as addition "membranes" of permeability $k_b$, hindering the transport of solute from the high concentration left cell half to the low concentration right cell half.

The boundary layer correlation is obtained through a combination of experimental studies and theoretical analyses. The experimental studies were obtained under actual permeable wall conditions using multiple solutes and track-etched permeable membranes from the Poretics Corporation (which possess well-defined cylindrical pores). The multiple solutes used were urea, sucrose, vitamin $B_{12}$, and horse skeletal myoglobin (Sigma M-0630). The theoretical results were obtained using three dimensional finite element analysis, which solved the basic conservation equations governing flow and mass transfer in the diffusion cell assuming both constant concentration and permeable wall conditions.

The combination of experimental and theoretical analyses approached the correlation in terms of solute transport, and not in terms of dissolution or electrolytic techniques. Others have earlier used dissolution or electrolytic techniques for cells of varying size and geometry, but these attempts have not derived precise enough determinations of $k_b$ to be useful for characterizing immunoisolation membranes in the present compact diffusion cells. This is true because, at least in part, and as the derived boundary layer correlation demonstrates, that the boundary condition operative during membrane transport is that of a permeable wall, and not the constant wall concentration condition present during solid dissolution or electro-deposition.

The derived boundary layer correlation is expressed in the dimensionless form:

$$Sh_b = \Phi(Sh_w) A Re^m Sc^n \tag{4}$$

where:

$Sh_b$, $Re$, $Sc$, and $Sh_w$ are, respectively, the boundary layer Sherwood, Reynolds, Schmidt, and wall Sherwood numbers:

$$Sh_b = k_b R_c / D \tag{5}$$

$$Re = \omega R_c R_s / \nu \tag{6}$$

$$Sc = \frac{\nu}{D} \tag{7}$$

$$Sh_w = \frac{2 k_m}{D / \sqrt{\nu / \omega}} \tag{8}$$

and where:

$\nu$ is the kinematic viscosity of the solute used.
$\omega$ is the angular velocity of the impeller.
$R_c$ is the radius of the diffusion chamber.
$R_s$ is the radius of the impeller.
$D$ is the free diffusivity of the solute in solution.
$A$, $m$, and $n$ are correlation parameters.

The function $\Phi(Sh_w)$ is an adjustment factor arising from the permeable wall boundary condition at the membrane surface.

The derived boundary layer correlation for the diffusion cell becomes:

$$Sh_b = 0.166 \Phi(Sh_w) Re^{0.671} Sc^{0.338} \tag{9}$$

$$\Phi(Sh_w) = 1 + \frac{0.13460}{1 + 0.95732 Sh_w^{1.0303}} \tag{10}$$

**(iii) Determining Actual Membrane Mass Transfer Coefficient $k_m$**

Using the boundary layer correlation expressed in Equation (9), the technician first calculates $k_b$ assuming that $\Phi$ $(Sh_w) = 1$, based upon the known values for Sc (Equation 7) and Re (Equation 6), given the diffusion cell geometry and operating conditions.

Next, the technician calculates an estimated $k_m$, using Equation 3, with $k_o$ derived using Equations 1 and 2, and the $k_b$ derived in the preceding paragraph.

The technician then uses the estimated $k_m$ to calculate $Sh_w$, using Equation 8.

The technician next uses the calculated $Sh_w$ to recalculate $\Phi(Sh_w)$ using Equation 10. The technician then recalculates a new estimated $k_b$ using the recalculated $\Phi(Sh_w)$, again using Equation 3 (with $k_o$ remaining unchanged). The technician then recalculates $Sh_w$ (using Equation 8) and $\Phi(Sh_w)$ (using Equation 10) and $k_b$ based upon the recalculated $\Phi(Sh_w)$ (using Equation 9), and so on until the successively recalculations of $k_b$ converge at a single value.

Using this convergent value of $k_b$, together with the measured value of $k_o$, the technician ultimately derives $k_m$, which represents the permeability for the membrane for the particular solute.

This test methodology ultimately yields $k_m$ for each solute in the multi-solute "cocktail" used in the diffusion cell; namely vitamin $B_{12}$; myoglobin; albumin; IgG; and tryptophan.

The following Example shows the use of the above described methodology for deriving $k_m$ for a given solute and membrane.

## EXAMPLE 1

The permeability of a 15% CA membrane to vitamin $B_{12}$ along with the other solutes described above was determined using the above described methodology.

Table 1 lists the solute and solution properties, cell geometry, and test operating conditions. Table 2 presents the resulting peak height data for the determination of $k_0$.

## Table 1

## Solute and Solution Properties, Cell Geometry, and Test Operating Conditions for Example 1

| PARAMETER | VALUE |
|---|---|
| Solute | Vitamin $B_{12}$ |
| Solvent | PBS |
| Test Temoerature, °C | 37 |
| Diffusivity, $cm^2$/sec | $3.94 \times 10^{-6}$ |
| Dynamic viscosity, $\mu$, poise | 0.00701 |
| Density, $g/cm^3$ | 0.9993 |
| Kinematic viscosity, $\nu$, $cm^2$/sec | 0.007016 |
| Cell radius, $R_c$, cm | 0.978 |
| Impeller Radius, $R_s$, cm | 0.902 |
| Left chamber volume, $V_1$, $cm^3$ | 3.620 |
| Right chamber volume, $V_2$, $cm^3$ | 3.275 |
| Harmonic Mean Volume, $V_H$, $cm^3$ | 3.439 |
| Sample volume, $V_s$, $cm^3$ | 0.100 |
| Impeller speed, rpm | 100 |
| | |
| | |
| Reynolds number | 1317 |
| Schmidt number | 1781 |

Table 2

| Chromatographic Peak Height Data for Determination of $k_o$ in Example 1 | | | | |
|---|---|---|---|---|
| Sampling Point (i) | Time (min) | $C_1$ | $C_2$ | LHS of Equation No. 1 |
| 1 | 30 | 5943 | 1214 | 0.400 |
| 2 | 60 | 5012 | 1966 | 0.797 |
| 3 | 120 | 4095 | 2808 | 1.652 |
| 4 | 300 | 3422 | 3302 | 3.855 |
| 5 | 1200 | 3165 | 3237 | 4.442 |

From these peak height data, a value for $C_{1,avg}$ ($t_0$) of 7018 is calculated in accordance with Equation (2) using the first three sampling points only. The left hand side (LHS) of Equation (1) shown in Table 2 is then obtained. Least squares analysis of the left hand side (Ms) of Equation (1) against time gives a slope of 0.0137 minutes$^{-1}$ with a coefficient of determinaticn of 0.9991 using only the first three time periods and forcing a zero intercept. The fourth and fifth time points are not used because $C_1$ and $C_2$ are experimentally indistinguishable, indicating that the system is in equilibrium. From this slope and the appropriate data from Table 1, an overall mass transfer coefficient ($k_0$) of

1.304 x $10^{-4}$ cm/sec is calculated. Using this value of $k_0$, $k_m$ is calculated using the iterative technique described above. The results are shown in Table 3. After three iterations, a constant value of $k_m$ of 1.72 is obtained.

Table 3

| Iterative Calculation of km for Example 1 with $k_0$ = 1.304 x $10^{-4}$ cm/sec *(All k-values are x $10^{-4}$)* | | | |
|---|---|---|---|
| Iteration | $Sh_w$ | $k_b$, cm/sec | $k_m$, cm/sec |
| 0 | 1.0000 | 10.402 | 1.740 |
| 1 | 1.0415 | 10.834 | 1.718 |

The value for $k_m$ derived in this Example 1 corresponds to the $k_m$ value shown in Table 4 for the 15% CA (Sample 1) membrane for vitamin $B_{12}$.

## IV. Formed-in-place Immunoisolation Boundaries

### A. Preparation of Materials

Permeable cast membrane structures were manufactured by casting in place cellulose acetate in weight percentages of 15%, 17%, and 20% upon the PTFE surface of the GORE-TEX membrane (W.L. Gore and Associates), using the formed-in-place techniques described earlier.

Control membranes consisted of a 0.4 μm PTFE membrane (Biopore, Millipore Corporation) laminated to a similar PTFE surface of the GORE-TEX membrane. This control membrane has been shown by Bauker et al. to support high levels of vascularization when implanted in animals and humans.

### B. Characterizing Membrane Permeability

Permeability $k_m$ measurements for the 15% CA; 17% CA; and 20% CA cast membrane structures and the control membrane were made using the diffusion cell apparatus and calculation techniaues described earlier.

The following Table 4 lists the derived permeabilities $k_b$ (in cm/sec x $10^{-4}$) for the cast membrane structures and the control membrane to the various solutes Vitamin $B_{12}$ ($B_{12}$) ; myoglobin (MYO) ; albumin (ALB); and immunoglobulin (IgG).

Table 4

| Membrane Permeability Results | | | | | |
|---|---|---|---|---|---|
| *(Values are in cm/sec x $10^{-4}$)* | | | | | |
| | | Solute | | | |
| Membrane | Sample | Vitamin $B_{12}$ | Myoglobin | Albumin | IgG |
| Control | 1 | 1.60 | 0.481 | 0.343 | 0.237 |
| | 2 | 1.27 | 0.336 | 0.234 | 0.160 |
| Cast, 15% CA | 1 | 1.72 | 0.307 | 0.0015 | 0.0004 |
| | 2 | 1.78 | 0.315 | 0.0007 | ND |
| Cast, 17% CA | 1 | 1.82 | 0.138 | ND | ND |
| | 2 | 1.27 | 0.129 | ND | ND |
| Cast, 20% CA | 1 | 1.06 | 0.042 | ND | ND |
| | 2 | 1.00 | 0.029 | ND | ND |
| Note:  ND means "not detected" at a sensitivity of about of $<10^{-8}$ cm/sec. | | | | | |

The permeability profile for the control membrane shows that it readily passes both large and small solutes, with the permeabilities $k_m$ being in direct proportion to solute size.

The permeability profiles for all CA cast membranes show that the CA cast membranes all exhibit high permeability to small solutes. However, the profiles reveal significant differences among them. Also, the transport of the larger test solutes is increasingly hindered as solute size increases. For the 15% CA cast membrane, the transport for IgG (160,000 D) is three orders of magnitude lower than that of $B_{12}$ (1333 D). For the 17% and 20% CA cast membranes, the permeabilities to both albumin and IgG are below the detection limit of about $10^{-8}$ cm/sec. Although the differences among the membranes in permeabilities to large solute could not be dectected, extrapolation of the small solute values suggests that there are also very great differences in large solute permeabilities among these three CA cast membrane structures.

### C. Tissue Preparation

Lung tissue from fetuses from day 14½ time-pregnant female ICR mice were used as the source of xenogeneic tissue. Lung tissue from fetuses from day 15½ time-pregnant Lewis rats were used as the source of isogeneic tissue. Lung tissue from isogeneic and xenogeneic sources were collected and processed separately.

The collected lung tissues were placed in sterile microfuge tubes and allowed to gravity settle. The excess media was removed. The lungs were minced into approximately 1 $mm^3$ pieces, washed with surgical media, and placed in a sterile centrifuge tubes.

### D. Preparation of Implantation Devices

The membranes were cut into small circles to fit titanium implantation chambers, like that shown in Fig. 1. The control membranes were wet briefly in absolute ethanol and placed in 70% ethanol overnight for sterilization. The cast CA membrane structures were wet by soaking for five minutes each in 50%, 70% and 95% ethanol, followed by overnight sterilization in 70% ethanol. All membranes were then washed three times in sterile saline (0.9% NaCl).

The titanium chambers were sterilized by autoclaving. The inner spacers were sterilized by overnight ethanol soak followed by sterile saline.

Ten microliters of gravity settled minced tissue were loaded into each chamber. The assembly and washed briefly two times in Dulbecco's phosphate buffered saline (D-PBS).

### E. Implantation

Devices containing isogeneic or xenogeneic tissue were implanted into the epididymal fat pads of male Lewis rats. Each animal received one device containing xenogeneic tissue and one device containing isogeneic tissue. The implants in half the animals were removed after three weeks. The implants in the remaining half were removed after six weeks. The removed implants were placed in 2% glutaraldehyde in Sorensen's buffer.

### F. Histology

Explanted devices were prepared for histology by standard methods and stained with hematoxylin and eosin. Sections were scored by three criteria:

(I) Tissue Survival within the Device (Tis) (1 = no survival to 6 = healthy, well differentiated epithelial tissue).
(ii) Host Response Outside the Device (Host) (1 = low level reaction to 6 = high reaction, macrophages and lymphocytes and plasma cells).
(iii) the Number of Host Vascular Structures within One Cell Layer (about 15 μm) of the Membrane-Host Tissue Interface (VS).

The following Table 5 lists the histology results.

Table 5

| Histology Results for Iso- and Xenograft Implants | | | | | | | |
|---|---|---|---|---|---|---|---|
| | | Isograft | | | Xenograft | | |
| Membrane | Weeks Implanted | Tissue | Host | VS | Tissue | Host | VS |
| Control | 3 | 4.5 | 2.5 | 100 | 1.5 | 4.7 | 17 |
| | 6 | 3.7 | 2.7 | 130 | 1 | 4.7 | 18 |

Table 5   (continued)

| Histology Results for Iso- and Xenograft Implants | | | | | | | |
| | | Isograft | | | Xenograft | | |
| Membrane | Weeks Implanted | Tissue | Host | VS | Tissue | Host | VS |
| Cast | 3 | 5.3 | 2.2 | 101 | 3.7 | 4.8 | 58.3 |
| 15% CA | 6 | 5.0 | 1.8 | 76 | 4.3 | 4.5 | 52 |
| Cast | 3 | 4.5 | 2.0 | 32 | 2.8 | 4.0 | 63.7 |
| 17% CA | 6 | 5.0 | 1.7 | 39 | 4.2 | 3.5 | 67.3 |
| Cast | 3 | 4.2 | 1.5 | 16 | 4 | 2.3 | 26.3 |
| 20% CA | 6 | 4.2 | 2.2 | 29.3 | 3.5 | 2.3 | 34.3 |

As Table 4 shows, the CA cast membranes are composed of identical materials, yet have markedly different permeabilities. As Table 5 shows, the CA cast membranes likewise demonstrated different in vivo host responses.

**(i) Isografts**

It has been previously demonstrated that isogeneic tissue survives well after implantation within the control membrane. Similarly, isografts survived in all three CA cast membranes.

Regarding host response, indicative of the presence or absence of an immune response immediately outside the implanted device, Table 5 shows low to moderate host response for all membranes.

However, although significant survival was observed, isografts did less well in the 20% CA cast membranes than in the control membrane, as demonstrated by a decreased cell density. These data indicate that the permeability profile of the 20% CA cast membranes may begin to somewhat restrict nutrient transport.

**(ii) Xenografts**

Previous studies and the data in Table 5 demonstrate that the control membrane does not protect xenografts. Moreover, xenografts encapsulated in the control membranes were surrounded by a severe host inflammatory cell reaction, obstructing vascularization near the host tissue-membrane interface.

By contrast, xenogeneic tissue survived in all CA cast membranes.

In the 15% CA cast membrane significant amounts of viable xenogeneic tissue were present at both three and six weeks. This tissue survived despite a significant host response outside the membrane, although this response was not a severe as that observed with the control membrane.

In the 17% and 20% CA cast membranes, the host response was significantly reduced. In the case of the 20% CA cast membrane, the host response for xenografts was little different than the host response for isografts in the control membrane. There was less surviving tissue in the 17% and 20% CA cast membranes, but in both cases surviving fibroblasts and epithelial cells were observed at both three and six weeks.

The results are consistent with the premise that host immune response to xenografts can be significantly ameliorated by changing the membrane permeability profile. The control membrane has high permeability for high molecular weight solutes and correspondingly demonstrated the highest local immune response. The 15% CA cast membrane has a significantly lower permeability for high molecular weight solutes, and exhibited a diminished, although still strong local immune response. The 20% CA cast membrane, on the other hand, had the lowest permeability for large molecular weight solutes, and also had a greatly diminished local immune response.

Even in the face of a strong host cellular response, xenograft survival was still significant in the 15% CA cast membrane. This result suggests that the membrane afforded protection against an incoming humoral host response, but nevertheless enabled the initiation of a local inflammatory reaction by the egress of xenogeneic antigens shed from tissue within the device. This result is consistent with the premise that the shed xenogeneic antigens that initiate the local reaction by egress from the device are smaller than the molecules of the immune system that destroy the xenografts by entering from outside the device.

The permeability profile of the 20% CA cast membrane prevented both the stimulation of a local response (by egress of xenogeneic antigens from the device) and the flux of host immune factors (from outside the device). However, regarding nutrient support, the permeability profile of the 20% CA cast membrane showed little difference between isograft and xenograft survival. Both cell types showed relatively poor survival, although living tissue was always present. This suggests that the permeability profile of the 20% CA cast membrane, though highly effective in ameliorating local immune response, may lie near the preferred boarder for providing nutrient transport to sustain cell survival, particular when in high cell densities are present.

Therefore, with regard to xenografts, there is a balance between decreasing membrane permeability to prevent host immune response and maintaining a desired permeability to promote optimal tissue survival. This balance is approached when the permeable boundary between the implanted xenogeneic cells and host tissue has a permeability profile including a permeability for solutes of molecular size generally equal to or less than vitamin $B_{12}$ of at least 0.1 $\times 10^{-4}$ cm/sec and a permeability for solutes of molecular size equal to or greater than IgG of less than 1.0 $\times 10^{-7}$ cm/sec. In a preferred implementation, the permeable boundary between the implanted xenogeneic cells and host tissue has a permeability profile including a permeability for solutes of molecular size generally equal to or less than vitamin $B_{12}$ of at least 0.5 $\times 10^{-4}$ cm/sec and a permeability for solutes of molecular size equal to or greater than IgG of less than 1.0 $\times 10^{-8}$ cm/sec.

Various features of the invention are set forth in the following claims.

## Claims

1. A permeable structure forming a chamber to hold living cells, the structure including a first permeable region surrounding at least a portion of the chamber having a conformation that, when implanted in host tissue, substantially blocks penetration of host cells into the chamber while permitting solute transport, a second permeable region overlying the first permeable region having a conformation that, when implanted in host tissue, forms a permeable interface with host tissue that permits solute transport, the first permeable region comprising a solution of polymer material formed in place in contact with the second permeable region.

2. A permeable structure forming a chamber to hold living cells, the structure including a first permeable region surrounding at least a portion of the chamber having a conformation that, when implanted in host tissue, substantially blocks penetration of at least host inflammatory cells into the chamber while permitting solute transport, a second permeable region overlying the first permeable region having a conformation that, when implanted in host tissue, forms a permeable interface with host tissue that permits solute transport, the first permeable region comprising a solution of polymer material formed in place in contact with the second permeable region.

3. A permeable structure forming a chamber to hold living cells, the structure including a first permeable region surrounding at least a portion of the chamber having a conformation that, when implanted in host tissue, substantially blocks penetration of host immunogenic factors into the chamber while permitting solute transport, a second permeable region overlying the first permeable region having a conformation that, when implanted in host tissue, forms a permeable interface with host tissue that permits solute transport, the first permeable region comprising a solution of polymer material formed in place in contact with the second permeable region.

4. A permeable structure forming a chamber to hold living cells, the structure including a first permeable region surrounding at least a portion of the chamber having a conformation that, when implanted in host tissue, substantially blocks penetration into the chamber by host immunogenic factors and penetration from the chamber by antigens shed by living cells held within the chamber, while also permitting solute transport, a second permeable region overlying the first permeable region having a conformation that, when implanted in host tissue, forms a permeable interface with host tissue that permits solute transport, the first permeable region comprising a solution of polymer material formed in place in contact with the second permeable region.

5. A permeable structure forming a chamber to hold living cells, the structure including a first permeable region surrounding at least a portion of the chamber, a second permeable region overlying the first permeable region and, when implanted in host tissue, forming an interface with host tissue, the first permeable region comprising a solution of polymer material formed in place in contact with the second permeable region, the structure having a permeability for solutes of molecular size generally equal to or less than vitamin $B_{12}$ of at least 0.1 $\times 10^{-4}$ cm/sec and a permeability for solutes of molecular size equal to or greater than IgG of less than 1.0 $\times 10^{-7}$ cm/sec.

6. A permeable structure according to claim 5 wherein the structure has a permeability for solutes of molecular size generally equal to or less than vitamin $B_{12}$ of at least 0.5 $\times 10^{-4}$ cm/sec and a permeability for solutes of molecular size equal to or greater than IgG of less than 1.0 $\times 10^{-8}$ cm/sec.

7. A permeable structure according to claim 1 or 2 or 3 or 4 or 5

wherein the second permeable region, when implanted in host issue, promotes the growth of host vascular structures adjacent the interface.

**8.** A permeable structure according to claim 1 or 2 or 3 or 4 or 5

wherein the second permeable region comprises an array of three dimensional strands having one dimension larger than the other two and, for the majority of strands, neither of the smaller dimensions exceeds about 5 $\mu$m.

**9.** A permeable structure according to claim 8

wherein the second permeable region comprises a membrane made from PTFE material free of a backing material.

**10.** A permeable structure according to claim 1 or 2 or 3 or 4 or 5 wherein the first permeable region comprises a cast solution of polymer material coagulated in place in contact with the second permeable region.

**11.** A permeable structure according to claim 1 or 2 or 3 or 4 or 5

wherein the polymer material of the first permeable region is selected from a group consisting essentially of cellulose acetate; poly(ethylene-co-vinyl alcohol); cuprammonium cellulose; poly(acrylonitrile); and acrylic copolymers.

**12.** A permeable structure according to claim 11 wherein the polymer material comprises cellulose acetate.

**13.** A permeable structure according to claim 11 wherein the polymer material comprises 15% to 20% by weight of cellulose acetate.

**14.** A permeable structure according to claim 1 or 2 or 3 or 4 or 5 and further including living cells in the chamber.

**15.** A permeable structure according to claim 1 or 2 or 3 or 4 or 5 and further including instructions teaching enclosure of living cells within the chamber and implantation of the permeable structure with enclosed living cells in host tissue.

**16.** A method for making a device for implanting in host tissue comprising the steps of

providing a permeable membrane having a conformation that permits solute transport, the permeable membrane having a surface,
applying a solution of polymer material in contact with the surface of the permeable membrane,
forming the applied polymer material in place on the permeable membrane, the applied polymer material forming a permeable layer having a conformation that, when implanted in host tissue, substantially blocks penetration of host cells while permitting solute transport through the permeable membrane, the permeable membrane and the permeable layer forming an integrated membrane structure, and
forming the integrated membrane structure into a device with the permeable layer enclosing a chamber to hold living cells and the permeable membrane positioned outside the chamber to form, when the device is implanted in host tissue, an interface with host tissue.

**17.** A method according to claim 16 wherein the step of applying the polymer material includes casting the solution of polymer material in contact with the permeable membrane surface, and wherein the step of forming the polymer material includes coagulating the cast polymer material in place on the permeable membrane.

**18.** A method according to claim 16 wherein the solution of polymer material is applied in contact substantially with the entirety of the surface of the permeable membrane.

**19.** A method according to claim 16 and further including the step of treating the surface of the permeable membrane with surfactant before the step of applying the polymer material, to thereby promote penetration of the polymer material into the surface of the permeable membrane.

**20.** A method according to claim 16 wherein the step of applying the polymer material includes the step of applying a polymer solution with a polymer material selected from a group consisting essentially of cellulose acetate; poly(ethylene-co-vinyl alcohol); cuprammonium cellulose; poly(acrylonitrile); and acrylic copolymers.

**21.** A method according to claim 20 wherein the polymer material comprises cellulose acetate.

22. A method according to claim 21 wherein the polymer material comprises 15% to 20% by weight of cellulose acetate.

23. A method according to claim 16 wherein the step of applying the polymer material includes casting the solution of polymer material in contact with the permeable membrane surface, and wherein the step of forming the polymer material includes coagulating the cast polymer material in place on the permeable membrane in a thermostated coagulation bath.

24. A method according to claim 23 and further including, after the step of coagulating, the step of washing the integral membrane structure.

25. A method according to claim 16 and further including, after the step of forming the applied polymer material, the step of treating the integral membrane structure with plasticizer.

26. A method according to claim 16 wherein the polymer material, after forming in place on the permeable membrane, forms a permeable layer having a conformation that, when implanted in host tissue, substantially blocks the penetration of at least host inflammatory cells.

27. A method according to claim 16 wherein the polymer material, after forming in place on the permeable membrane, forms a permeable layer having a conformation that, when implanted in host tissue, substantially blocks the penetration of host immunogenic factors.

28. A method according to claim 16 wherein the polymer material, after forming in place upon the permeable membrane, forms a permeable layer having a conformation that, when implanted in host tissue, substantially blocks the penetration of both host immunogenic factors and antigens shed by implanted cells.

29. A method according to claim 16

wherein, in the step of providing a permeable membrane, a microporous membrane made from PTFE material is provided having a backing material, and
and further including, after the step of forming the applied polymer material in place on the permeable membrane to form the integral membrane structure, the step of removing the backing material.

30. A method for making a device for implanting in host tissue comprising the steps of

providing a permeable membrane having a conformation that permits solute transport, the permeable membrane having a surface,
locating the permeable membrane on a casting plate with the surface of the permeable membrane facing away from the casting plate,
casting a solution of polymer material in contact with the surface of the permeable membrane while the permeable membrane is located on the casting plate, the casting being performed while the casting plate is at a determinable temperature,
bringing a coagulation bath to a temperature different than the determinable temperature of the casting plate, immersing the casting plate with the cast polymer material in place on the permeable membrane into the coagulation bath to coagulate the cast polymer material into a permeable layer having a conformation that, when implanted in host tissue, substantially blocks penetration of host cells while permitting solute transport through the permeable membrane, the permeable membrane minimizing the temperature gradient across the cast polymer material within the coagulation bath, forming an integrated membrane structure on the casting plate,
removing the integrated membrane structure from the casting plate, and
forming the integrated membrane structure into a device with the permeable layer enclosing a chamber to hold living cells and the permeable membrane positioned outside the chamber to form, when the device is implanted in host tissue, an interface with host tissue.

31. A method according to claim 30
wherein, in the step of providing a permeable membrane, a microporous membrane made from PTFE material is provided.

32. A method according to claim 30 or 31 wherein the step of casting includes the step of creating the polymer solution with a polymer material selected from a group consisting essentially of cellulose acetate; poly(ethylene-co-vinyl alcohol); cuprammonium cellulose; poly(acrylonitrile); and acrylic copolymers.

33. A method according to claim 32 wherein the polymer material comprises cellulose acetate.

34. A method of implanting living cells in host tissue comprising the steps of

making a device having a chamber for implanting in host tissue the following the method defined in claim 16 or 30,
placing living cells in the chamber of the device, and
implanting the device with living cells in the chamber in host tissue.

35. A method for implanting living xenogeneic cells in host tissue comprising the steps of

placing the living xenogeneic cells in a permeable structure forming a chamber to hold the living xenogeneic cells, the structure including a first permeable region surrounding at least a portion of the chamber, a second permeable region overlying the first permeable region and, when implanted in host tissue, forming an interface with host tissue, the first permeable region comprising a cast solution of polymer material coagulated in place in contact with the second permeable region, the structure having a permeability for solutes of molecular size generally equal to or less than vitamin $B_{12}$ of at least $0.1 \times 10^{-4}$ cm/sec and a permeability for solutes of molecular size equal to or greater than IgG of less than $1.0 \times 10^{-7}$ cm/sec, and
implanting the permeable structure holding the living xenogeneic cells in host tissue.

36. A method for implanting living xenogeneic cells in host tissue comprising the steps of

placing the living xenogeneic cells in a permeable structure forming a chamber to hold the living xenogeneic cells, the structure including a first permeable region surrounding at least a portion of the chamber, a second permeable region overlying the first permeable region and, when implanted in host tissue, forming an interface with host tissue, the first permeable region comprising a cast solution of polymer material coagulated in place in contact with the second permeable region, the structure having a permeability for solutes of molecular size generally equal to or less than vitamin $B_{12}$ of at least $0.5 \times 10^{-4}$ cm/sec and a permeability for solutes of molecular size equal to or greater than IgG of less than $1.0 \times 10^{-8}$ cm/sec, and
implanting the permeable structure holding the living xenogeneic cells in host tissue.

FIG.1

INSTRUCTIONS
FOR USE

FIG.2

FIG.3

FIG.4

FIG.5

## FIG.6

## FIG.7

FIG.8

FIG.9

FIG.10

FIG.11

FIG.12

58

52

44

20 40 42

18

48

46

56

FIG.13

EP 0 747 046 A2

FIG.14